# EUROPÄISCHE PATENTSCHRIFT

(11) **EP 1 894 538 B1**
(45) Veröffentlichungstag und Bekanntmachung des Hinweises auf die Patenterteilung: **04.11.2009**
(21) Anmeldenummer: 06018341.5
(22) Anmeldetag: 01.09.2006
(51) Int. Cl.: A61B 19/00

(54) **Verfahren und Vorrichtung zum Bestimmen der Lage von Beckenebenen**
Method and device for determining the position of pelvic planes
Procédé et appareil pour la détermination de la position des plans pelviens

(43) Veröffentlichungstag der Anmeldung: 05.03.2008
(73) Patentinhaber: BrainLAB AG, 85622 Feldkirchen (DE)
(72) Erfinder: Lachner, Rainer, 81547 München (DE); Steinle, Wolfgang, 80337 München (DE); Vilsmeier, Stefan, 80539 München (DE)
(74) Vertreter: Schwabe - Sandmair - Marx

(56) Entgegenhaltungen:
- EP-A- 1 611 863
- WO-A-02/062248
- WO-A-2005/000140
- US-A1- 2003 153 829
- US-A1- 2004 102 792

## Beschreibung

Die vorliegende Erfindung bezieht sich auf ein Verfahren und eine Vorrichtung zum Bestimmen der Lage von Hüft- oder Beckenebenen, insbesondere auf das Bestimmen der Lage der frontalen oder vorderen Beckenebene aus Fluoro-Aufnahmen, so genannten Fluoro-Shots.

Die Bestimmung der Lage von die Position oder Ausrichtung des Beckens charakterisierenden Ebenen, insbesondere die Bestimmung der Lage der Mittsagittal-Ebene und die Lage der vorderen oder frontalen Beckenebene, welche auf vier Punkten des Beckens, nämlich den zwei Pubis-Punkten und zwei Spina-Punkten, aufliegt, ist zum Beispiel in der Hüftchirurgie von großer Bedeutung. Wenn zum Beispiel eine neue Gelenkpfanne eingesetzt werden soll, ist deren exakte Positionierung unter anderem von der Lage der Mittsagittal-Ebene und der frontalen Beckenebene abhängt. Insbesondere ist es für das Einsetzen eines künstlichen Hüftgelenkes erforderlich, die Ausrichtung des acetabularen Beckenimplantats (pelvic acetabular implant) in Bezug auf diese beiden ortogonal zueinander liegenden Ebenen, die Mittsagittal-Ebene und die frontale Beckenebene, zu messen oder zu bestimmen.

Je nachdem welche Bilddaten verfügbar sind, können diese Ebenen auf unterschiedliche Art bestimmt werden. So können, wenn die Navigation zum Beispiel auf einem CT Scan basiert, diese Ebenen in dem CT Volumen definiert werden. Weil der CT-Datensatz in Bezug auf den Patienten registriert ist, können diese Daten von den CT-Koordinaten in Patienten-Koordinaten übertragen oder umgerechnet werden. Wenn eine Navigation ohne bildgebende Verfahren durchgeführt wird, werden diese Ebenen gewöhnlich direkt auf dem Patienten unter Verwendung eines Pointers definiert.

Aus der EP 1 570 800 A1 der Anmelderin ist ein Verfahren und eine Vorrichtung zum Bestimmen einer Symmetrieebene eines dreidimensionalen Objektes und insbesondere zum Bestimmen der Mittsagittal-Ebene einer Hüfte basierend auf zwei Röntgen- oder Fluoro-Aufnahmen bekannt, wobei die Lehre der EP 1 570 800 A1 bezüglich der Bestimmung dieser Symmetrie- oder Mittsagittal-Ebene in diese Anmeldung aufgenommen wird.

Aus der US 2004/0102792 A1 und der WO 2004/089192 A2 sind Verfahren zum Bestimmen der Position eines Beckenknochens bekannt, wobei mittels eines Pointers Positionen von Punkten an einem Patienten ermittelt werden.

Aus der WO 02/062248 A1 ist bekannt, dass drei Landmarken einer Hüfte zum Beispiel durch Palpation oder einem Pointer identifiziert werden sollen, um die frontale Beckenebene zu definieren. Die so aufgefundenen Punkte werden zum Beispiel durch laterale Röntgenbilder präzisiert.

Es ist eine Aufgabe der Erfindung ein Verfahren und eine Vorrichtung vorzuschlagen, welche die Bestimmung der Lage der vorderen oder frontalen Beckenebene basierend auf mindestens zwei Röntgen- oder Fluoro-Aufnahmen ermöglicht.

Diese Aufgabe wird durch die Gegenstände der unabhängigen Ansprüche gelöst. Vorteilhafte Ausführungsformen ergeben sich aus den abhängigen Patentansprüchen.

Zur Bestimmung der Lage oder Position einer Hüfte im Raum können die Mittsagittal-Ebene und die frontale Beckenebene verwendet werden, wobei der so genannte Pubis-Punkt auf beiden Ebenen liegt.

Bei einem erfindungsgemäßen Verfahren zum Bestimmen der Lage von charakteristischen Beckenebenen und insbesondere zum Bestimmen der Lage der frontalen Beckenebene (Frontal Pelvic Plane) im Raum und/oder relativ zum Beckenknochen bzw. relativ zur Hüfte wird durch ein bekanntes Verfahren die Lage der Mittsagittal-Ebene bestimmt, wobei das in der EP 1 570 800 A1 beschriebene Verfahren oder andere bekannte Verfahren zum Beispiel unter Verwendung von Pointern verwendet werden können. Weiterhin wird die Position des Pubis-Punktes, also des Punktes auf dem Pubis-Knochen bestimmt, der am weitesten vorsteht, also die anterior am weitesten herausragende Position hat. Dieser Punkt kann zum Beispiel durch Röntgen- oder Fluoro-Aufnahmen und Bestimmen der Position des den Pubis-Punkt bildenden, charakteristisch vorstehenden Bereiches oder durch Ertasten zum Beispiel mittels eines Pointers bestimmt werden.

Sofern in dieser Anmeldung von Position oder Lage eines Punktes, einer Geraden oder einer Ebene gesprochen wird, kann darunter entweder die Position oder Lage des Punktes, der Geraden oder der Ebene im Raum oder auch relativ zur Hüfte oder dem Beckenknochen verstanden werden. Weiterhin wird davon ausgegangen, dass alle Röntgen- oder fluoroskopischen Bilder "kalibriert" sind, das heißt es ist bekannt aus welcher Richtung und/oder unter welchem Winkel relativ zum abgebildeten Objekt die jeweilige Abbildung erzeugt wurde, so dass zum Beispiel eine in dem jeweiligen Bild gezeigte Linie eine Struktur ist, die in der (Rück-) Projektionsebene der jeweiligen Abbildungsvorrichtung liegt.

Nicht erfindungsgemäß wird basierend auf mindestens einer kalibrierten Rönten- oder Fluoro-Aufnahme die Position oder Lage der frontalen Beckenebene wie nachfolgend beschrieben bestimmt, wobei die Tatsache ausgenutzt wird, dass die frontale Beckenebene senkrecht auf der zuvor bestimmten Mittsagittal-Ebene liegt und durch den Pubis-Punkt, sowie durch den Beckenkamm (iliac crista) geht.

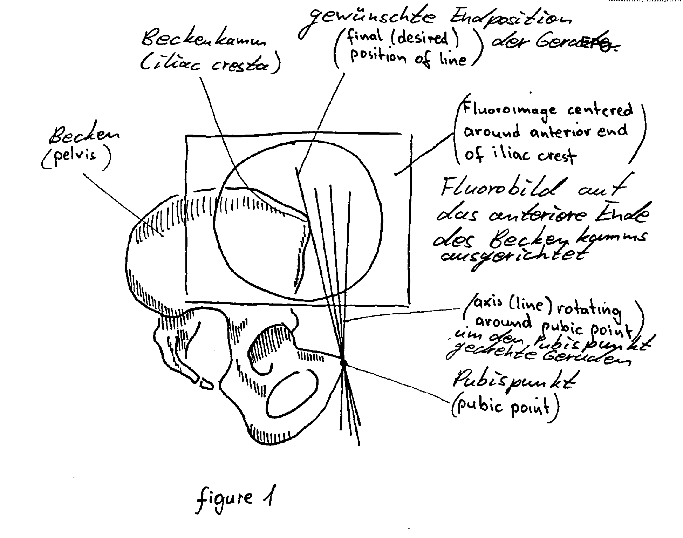

Falls es möglich ist ein laterales Fluorobild aufzunehmen, bei welchem der Projektionsmittelpunkt nahe bei oder sogar auf der frontalen Beckenebene liegt, kann die Lage der frontalen Beckenebene einfach aus dieser einzigen Aufnahme oder diesem Bild dadurch bestimmt werden, dass eine Gerade oder Linie in dem Fluorobild um die Projektion des Pubis-Punktes so gedreht wird, dass diese den Umriss oder die Kontur des Beckens, insbesondere des Beckenkammes oder Crista iliaca berührt, wie in Figur 1 gezeigt. Die frontale Beckenebene ist dann einfach die Rückprojektionsebene dieser Linie ausgehend von der gedrehten Endposition, bei welcher diese Linie durch den Pubis-Punkt und den Beckenkamm geht.

In der Praxis tritt bei der Aufnahme des Fluorobildes häufig eine kleine Abweichung auf, so dass die Mittsagittal-Ebene und die Rückprojektionsebene nicht mehr genau senkrecht aufeinander stehen. In diesem Fall wird die Rückprojektionslinie durch Drehen um die besagte Linie derart korrigiert, dass sie senkrecht auf der Mittsagittal-Ebene steht.

Diese Drehung kann entweder automatisch oder durch einen Benutzer durchgeführt werden, wozu zum Beispiel eine graphische Benutzeroberfläche oder ein graphisches Benutzer-Interface vorgesehen sein kann.

Falls kein laterales Fluoro-Bild aufgenommen werden kann, da zum Beispiel der Patient so gelagert ist, dass er hierzu gedreht werden müsste, was häufig vermieden werden sollte, oder zu viel für die Aufnahme ungünstig angeordnetes Fettgewebe aufweist, kann erfindungsgemäß die Lage der frontalen Beckenebene dadurch bestimmt werden, dass zwei Röntgen- oder Fluoro-Aufnahmen aus verschiedenen Aufnahme- oder Blickrichtungen durchgeführt werden. Wiederum wird in den jeweiligen Aufnahmen jeweils eine Linie durch den Pubis-Punkt gelegt und so lange gedreht, bis diese Linie an einer Kontur oder Umrandung des Beckenknochens anliegt. Durch eine Rückprojektion dieser beiden an zwei Punkten des Beckenknochens anliegenden Linien werden zwei Ebenen erhalten, welche sich in einer Linie oder Geraden schneiden, welche auf der frontalen Beckenebene liegt. Wenn diese so ermittelte Schnittlinie an der Mittsagittal-Ebene gespiegelt wird, wird eine andere Linie auf der frontalen Beckenebene erhalten, wobei die frontale Beckenebene durch diese beiden Linien eindeutig bestimmt ist.

Vorzugsweise können, wenn kein laterales Fluoro-Bild erzeugt werden kann, zur Bestimmung der Lage der Mittsagittal-Ebene zum Beispiel gemäß der Lehre der EP 1 570 800 A1 die beiden gleichen Röntgen- oder Fluoro-Aufnahmen verwendet werden, wie zur Bestimmung der Lage der frontalen Beckenebene. Jedoch ist es auch möglich, nur eines oder auch andere Fluoro-Bilder optional auch in Kombination mit bereits vorhandenen Bildern oder Aufnahmen zu verwenden.

Der Patient und vorzugsweise der Beckenknochen kann mit mindestens einem Marker oder Marker-System, wie zum Beispiel einem Referenzstern, verbunden sein, um zum Beispiel in Kombination mit einem bekannten Tracking-System den Beckenknochen lokalisieren oder tracken zu können. Ebenso ist es möglich, dass der Patient und vorzugsweise der Beckenknochen zum Beispiel mittels eines bekannten Gestells oder Frames ortsfest gelagert ist, wobei beispielsweise an dem Gestell oder einer damit verbundenen Vorrichtung, wie zum Beispiel einem Operationstisch, mindestens ein Marker oder eine Markeranordnung verbunden ist.

Durch die Verwendung des erfindungsgemäßen Verfahrens ist es somit möglich, alle zum Positionieren eines künstlichen Hüftgelenkes erforderlichen Daten und insbesondere die Position oder Lage des Beckenknochens oder dessen charakteristischen Ebenen im Raum einfach zu bestimmen, ohne dass zum Beispiel ein Punkt auf der Hüfte manuell zum Beispiel mittels eines Pointers erfasst werden muss. Das Verfahren kann zum Beispiel basierend auf nur zwei Fluoro-Aufnahmen durchgeführt werden. Insbesondere ist das erfindungsgemäße Verfahren vorteilhaft, da kein Asis-Punkt, welcher nicht einfach zu identifizieren ist, bestimmt werden muss, um die Lage der frontalen Beckenebene zu ermitteln.

Bevorzugt werden die mindestens zwei zweidimensionalen Abbildungen des Beckenknochens durch Aufnahmen zum Beispiel mittels Röntgenstrahlen oder sogenannte Fluoroschüsse erzeugt, wobei die Aufnahmen vorteilhaft aus unterschiedlichen Winkeln erfolgen. Vorzugsweise werden die Aufnahmen des Beckens aus zwei verschiedenen Richtungen durchgeführt, wobei die Projektionszentren mit dem Objekt vorzugsweise einen Winkel von 5° bis 30°, also zum Beispiel einen Winkel von 10° einschließen. Es können auch mehrere Aufnahmen des Objektes aus unterschiedlichen Richtungen durchgeführt werden, wobei beispielsweise mit jeweils zwei zweidimensionalen Abbildungen des dreidimensionalen Objektes das oben beschriebene Verfahren durchgeführt werden kann, um die Genauigkeit bei der Ermittlung der Mittsagittal-Ebene und der frontalen Beckenebene des Beckens zu erhöhen. Vorteilhaft kann unmittelbar an dem abzubildenden Beckenknochen ein Referenzstern oder Marker angebracht sein, um das räumliche Verhältnis des Objektes relativ zu den Projektionszentren und zugeordneten Abbildungsebenen zu ermitteln, wobei diese Lokalisationsdaten zur Durchführung des erfindungsgemäßen Verfahrens verwendet werden können.

Weiterhin bezieht sich die Erfindung auf ein Computerprogramm, welches, wenn es in einen Computer geladen wird oder auf einem Computer läuft, einen oder mehrere der oben beschriebenen Verfahrensschritte durchführt, sowie auf eine Programmspeichermedium oder Computerprogrammprodukt mit einem solchen Programm.

Nach einem weiteren Aspekt bezieht sich die Erfindung auf eine Vorrichtung zur Bestimmung der Lage der frontalen Beckenebene mit zum Beispiel einem C-Bogen mit mindestens einem Projektionszentrum, wie zum Beispiel einer Strahlenquelle und mindestens einer Projektionsebene, mit welchen eine zweidimensionale Abbildung des dreidimensionalen Objektes erzeugt werden kann. Durch Drehung des Projektionszentrums und der Projektionsebene um das Becken oder durch Verwendung eines weiteren Projektionszentrums oder einer weiteren Projektionsebene kann mindestens eine weitere zur Durchführung des erfindungsgemäßen Verfahrens vorteilhafte zweidimensionale Abbildung des dreidimensionalen Objektes erzeugt werden, wobei erfindungsgemäß mindestens ein Marker oder Referenzstern vorgesehen ist, welcher direkt oder indirekt an dem Objekt angebracht wird, wodurch das Lageverhältnis des Objektes relativ zu dem mindestens einen Projektionszentrum und/oder der mindestens einen Projektionsebene ermittelt werden kann.

Weiterhin ist erfindungsgemäß eine Recheneinheit vorgesehen, welche ein und bevorzugt mindestens zwei zweidimensionale Abbildungsdaten des dreidimensionalen Objektes und die räumlichen Lagedaten des Objekts, des Projektionszentrums und der Abbildungsebene erhält und ein wie oben beschriebenes Verfahren ausführen kann.

Nachfolgend wird die Erfindung anhand eines bevorzugten Ausführungsbeispieles beschrieben. Es zeigen:
- Figur 1: ein Verfahren zum Bestimmen der frontalen Beckenebene mittels eines lateralen Fluoro-Bildes; und
- Figur 2: eine Ausführungsform der Erfindung zur Bestimmung der Lage der frontalen Beckenebene mittels zweier aus unterschiedlichen Richtungen aufgenommenen Fluoro-Bildern.

Figur 1 zeigt eine laterale oder Seitenaufnahme eines Beckenknochens, wobei eine Gerade durch den Pubis-Punkt gelegt und solange gedreht wird, bis sie in ihrer Endposition an dem Beckenkamm (Crista iliaca) anliegt. Da in der Seitenaufnahme die Mittsagittal-Ebene auch gleichzeitig die Bildebene ist und somit die Rückprojektion der Geraden eine Ebene erzeugt, die senkrecht auf der Mittsagittal-Ebene steht und sowohl durch den Pubis-Punkt, als auch durch den Beckenkamm verläuft, ist die durch diese Rückprojektion gefundene Ebene auch gleichzeitig die frontale Beckenebene, so dass deren Lage im Raum oder relativ zum Beckenknochen bestimmt ist.

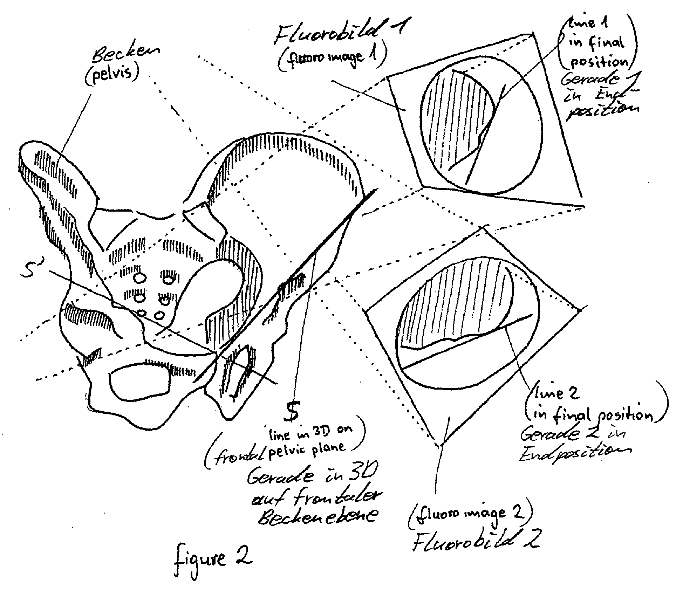

Figur 2 zeigt ein Verfahren, bei welchem keine laterale Aufnahme des Beckenknochens durchgeführt wurde und zwei Aufnahmen aus geeigneten unterschiedlichen Richtungen erzeugt wurden. Wie in Figur 1 beschrieben, wird für beide Fluoro-Bilder jeweils eine Gerade durch den Pubis-Punkt gelegt und solange gedreht, bis diese Gerade an einer Kontur des Beckenknochens in dem jeweiligen Fluoro-Bild anliegt. Durch die Rückprojektion der jeweiligen so ermittelten Geraden in Endposition werden zwei Ebenen erhalten, welche sich in der mit S bezeichneten Schnittgeraden treffen. Diese Schnittgerade S wird an der zuvor ermittelten Mittsagittal-Ebene gespiegelt, so dass die gespiegelte Gerade S' erhalten wird. Die durch die beiden Geraden S und S' definierte Ebene ist die frontale Beckenebene, deren Lage im Raum oder relativ zum Beckenknochen aus zwei aus unterschiedlichen Richtungen gemachten Fluoro- oder Röntgen-Aufnahmen bestimmt wurde.

## Patentansprüche

1. Verfahren zum Bestimmen der Lage der frontalen Beckenebene, wobei die Lage der Mittsagittal-Ebene bestimmt wird, die Position eines Pubis-Punktes ermittelt wird, wobei mindestens zwei Röntgenaufnahmen des Beckenknochens aus unterschiedlichen Richtungen gemacht werden, jeweils eine Gerade in jedem der erzeugten Aufnahmebilder bestimmt wird, welche durch die Abbildung des Pubis-Punktes verläuft und an einer Kontur des abgebildeten Beckenknochens anliegt, die durch die Rückprojektion der so ermittelten Geraden erzeugten Ebenen eine Schnittgerade S bilden, welche an der Mittsagittal-Ebene gespiegelt wird, um die Gerade S' zu erhalten, wobei die frontale Beckenebene durch die Geraden S und S' definiert wird.

2. Verfahren nach dem vorhergehenden Anspruch, wobei an dem Beckenknochen und/oder dem Patienten mindestens ein Marker angebracht ist und mit einem Navigationssystem die Lage des Beckenknochens im Raum ermittelt wird.

3. Verfahren nach einem der vorhergehenden Ansprüche, wobei der Beckenknochen durch ein Gestell in einer Position fixiert wird und ein Marker an dem Gestell oder einer mit dem Gestell verbundenen Struktur befestigt ist.

4. Verfahren nach einem der vorhergehenden Ansprüche, wobei mehrere Fluorobilder des Beckens erzeugt werden und aus jeweils zwei Fluorobildem die Lage der frontalen Beckenebene ermittelt wird.

5. Computerprogramm, welches, wenn es in einen Computer geladen wird oder auf einem Computer läuft, ein in den vorangehenden Ansprüchen beschriebenes Verfahren durchführt.

6. Programmspeichermedium oder Computerprogrammprodukt mit einem Programm nach dem vorhergehenden Anspruch.

7. Vorrichtung zur Bestimmung der Lage der frontalen Beckenebene aus mindestens einer zweidimensionalen Aufnahme des Objektes mit mindestens einem Projektionszentrum und mindestens einer dem Projektionszentrum zugeordneten Projektionsebene, mindestens einem Marker, welcher mit dem dreidimensionalen Objekt verbunden werden kann und mit einer Recheneinheit, welcher durch die Marker ermittelte Positionsdaten des Objektes und die Aufnahmedaten des dreidimensionalen Objektes in der mindestens einen Projektionsebene zugeführt werden und welche daraus die Lage der frontalen Beckenebene nach einem der Ansprüche 1 bis 4 berechnet.

8. Vorrichtung nach dem vorhergehenden Anspruch, wobei das Projektionszentrum eine Strahlenquelle, insbesondere eine Röntgenstrahlenquelle ist.

## Claims

1. Method of determining the position of the frontal pelvic plane, whereby the position of the mid-sagittal plane is defined, the position of a pubic point is determined, at least two X-ray images of the pelvic bone are taken from different directions, a respective line is defined in each of the resultant images which extends through the image of the pubic point and lies on a contour of the imaged pelvic bone, the planes generated by back-projecting the lines determined in this manner form an intersecting line S which is mirrored on the mid-sagittal plane in order to obtain the line S', and the frontal pelvic bone is defined by the lines S and S'.

2. Method as claimed in the preceding claim, whereby at least one marker is attached to the pelvic bone and/or to the patient and the position of the pelvic bone in space is determined by means of a navigation system.

3. Method as claimed in one of the preceding claims, whereby the pelvic bone is fixed in a position by means of a frame and a marker is attached to the frame or to a structure connected to the frame.

4. Method as claimed in one of the preceding claims, whereby several fluoro-images of the pelvis are taken and the position of the frontal pelvic plane is determined from two respective fluoro-images.

5. Computer programme which runs a method as described in the preceding claims when loaded onto a computer or run on a computer.

6. Programme storage medium or computer programme product with a programme as claimed in the preceding claim.

7. Device for determining the position of the frontal pelvic plane from at least one two-dimensional image of the object with at least one projection centre and at least one projection plane assigned to the projection centre, at least one marker which can be connected to the three-dimensional object, and with a computer unit which can be moved in the at least one projection plane on the basis of the position-related data of the object determined by the markers and the image data of the three-dimensional object and which calculates the position of the frontal pelvic plane therefrom as claimed in one of claims 1 to 4.

8. Device as claimed in the preceding claim, whereby the projection centre is an irradiation source, in particular an X-ray radiation source.

## Revendications

1. Procédé pour déterminer la position du plan pelvien antérieur dans lequel on détermine la position du plan sagittal médian, la position d'un point du pubis, dans lequel on réalise au moins deux radiographies de l'os du bassin à partir de différentes directions, on détermine chaque fois une droite dans chacune des images produites qui passe par la reproduction du point du pubis et s'applique contre un contour de l'os du bassin reproduit, les plans produits par la rétroprojection des droites ainsi déterminées forment une droite d'intersection (S) qui est réfléchie sur le plan sagittal médian, afin d'obtenir la droite (S'), le plan pelvien antérieur étant défini par les droites (S, S').

2. Procédé selon la revendication 1, dans lequel on place au moins un repère sur l'os du bassin et/ou le patient, et on détermine la position dans l'espace de l'os du bassin avec un système de navigation.

3. Procédé selon l'une quelconque des revendications 1 ou 2, dans lequel l'os du bassin est fixé dans une position par un bâti et un repère est fixé sur le bâti ou sur une structure reliée au bâti.

4. Procédé selon l'une quelconque des revendications 1 à 3, dans lequel on réalise plusieurs images fluo du bassin et on détermine la position du plan pelvien antérieur dans chaque cas à partir de deux images fluo.

5. Programme d'ordinateur qui lorsqu'il est chargé dans un ordinateur ou tourne dans un ordinateur, exécute un procédé décrit dans les revendications 1 à 4.

6. Support de mémoire de programme ou produit de programme d'ordinateur avec un programme selon la revendication 5.

7. Dispositif pour déterminer la position du plan pelvien antérieur à partir d'au moins une prise de vue bidimensionnelle de l'objet avec au moins un centre de projection et au moins un plan de projection associé au centre de projection, au moins un repère qui peut être relié à l'objet tridimensionnel et avec une unité informatique à laquelle sont amenées des données de position de l'objet, déterminées par les repères, ainsi que les données de prise de vue de l'objet tridimensionnel dans le au moins un plan de projection, et qui à partir de ceci calcule la position du plan pelvien antérieur selon l'une des revendications 1 à 4.

8. Dispositif selon la revendication 7, dans lequel le centre de projection est une source de rayonnement, en particulier une source de rayons X.
